Europäisches Patentamt

European Patent Office

Office européen des brevets

(19)

(11) Publication number : **0 147 100 B2**

(12) # NEW EUROPEAN PATENT SPECIFICATION

(45) Date of publication of the new patent specification : **10.02.93 Bulletin 93/06**

(51) Int. Cl.⁵ : **G02C 13/00, A61L 2/18**

(21) Application number : **84308448.4**

(22) Date of filing : **05.12.84**

(54) **Contact lenses.**

(30) Priority : **06.12.83 GB 8332489**

(43) Date of publication of application :
**03.07.85 Bulletin 85/27**

(45) Publication of the grant of the patent :
**27.09.89 Bulletin 89/39**

(45) Mention of the opposition decision :
**10.02.93 Bulletin 93/06**

(84) Designated Contracting States :
**AT BE CH DE FR GB IT LI LU NL SE**

(56) References cited :
**EP-A- 0 082 798**
**EP-A- 0 124 461**
**EP-A- 0 139 994**
**GB-A- 1 464 333**
**GB-A- 1 484 972**
**US-A- 3 912 451**

(73) Proprietor : **CIBA-GEIGY AG**
**Klybeckstrasse 141**
**CH-4002 Basel (CH)**

(72) Inventor : **Kay, Brian**
**17 Long Close, Farnham Common**
**Slough, Buckinghamshire (GB)**

EP 0 147 100 B2

## Description

The present invention relates to contact lenses, and in particular to prophylatic methods for cleaning and disinfecting contact lenses, and to tablets for use in the method.

Contact lenses require frequent cleaning due to the build-up of deposits. The source of most deposits is the tear fluid, although poor hygiene practice can allow transfer of dirt, cosmetics and other extraneous contaminatants to the lens surface. Since hard and flexible lenses are essentially hydrophobic they have no strong tendency to accumulate polar deposits such as proteins or inorganic compounds. This is reflected in the comparative ease with which the lenses are cleaned. However, soft lenses and gas permeable lenses, by nature of their polar surfaces, are prone to extensive deposition of tear fluid components.

Since most deposits originate from the tear fluid, and the composition of tear fluid is dependent on the individual (and even then is not constant), deposit formation will vary from patient to patient.

The implications of dirty contact lenses and lens deposits are numerous. For example, they reduce the disinfecting action of preservatives, increasing the risk of infection; they alter the extent of the hydration and hence physical parameters of the lens; they reduce visual acuity; they cause chronic discomfort; they promote red-eye syndromes; they reduce surface wetting; they shorten tear film break-up time, causing formation of dry spots; they initiate lens discolouration; they decrease lubrication of the lens surface; they increase production of tear fluid; they decrease contact lens wearing time; and ultimately they cause discontinuation of use.

Since contact lenses are cleaned outside the eye, stronger agents can be used than might otherwise be tolerated. However, there is always a possibility that a misinformed patient may directly transfer the cleaning agent to his eye. Thus a perequisite of all cleaning solutions is that should be no irreversible occular insult if accidentally instilled into the eye.

The usual method of cleaning hydrophobic lenses is with a surfactant solution: the preferred agents are non-ionic and amphoteric surfactants, which work best in alkaline pH and act to emulsify lipids and solubilise debris, although certain cationic and anionic detergents have also been used.

For cleaning soft (hydrophilic) lenses, the available can be classified as surfactant cleaners of variable strenght, enzyme cleaners and oxidative cleaners. In general, surfactants used daily provide a prophylactic cleaning service but cannot remove denatured protein deposits without excessive abrasion. Enzymes can be used to digest specific forms of deposit, but lack action against non-protein/non-lipid deposits, have high cast and give problems with patients reactions, leading to patient non-compliance.

Oxidation usually involves heating the lens, although some are effective at room temperature over longer periods. There is often a swelling of soft lenses during oxidative treatments. Moreover, several of the oxidative methods are available only to practitioners and thus cannot be adopted by patients as part of a regular lens care regime.

Two oxidative cleaning methods are currently available for lay use. One method utilises chlorine as the oxidizing agent, donated for example by dichloroisocyanurate dihydrate. The tablets also contain sodium chloride, citric acid and sodium bicarbonate. The tablet is placed in normal saline with the lenses, producing effervesence and realising the chlorine. After 10 minutes, the lenses are removed, rinsed and boiled for 15 minutes in saline. After repeating, the lenses are left in their normal soaking solution overnight.

Unfortunately, lenses which are only partially cleaned have tendency to be stained orange, due to the reaction of chlorine with chlorhexidine digluconate bound to the lens.

The other oxidative method is primarily a disinfection system, but it also has mild cleaning properties, because of its oxidative action. It involves the use of 3% hydrogen peroxide and normal saline in two separate cases. The lenses are initially rubbed with the peroxide solution by hand and then placed in one case with the peroxide solution (within a basket device) for 20 minutes. After cleaning, the basket is transferred to a second case, containing the saline and a plastic disc coated with platinum. This is left for a minimum of 4 hours, during which the remaining $H_2O_2$ is deactivated to form water. The need to follow carefully the overall procedure is apparent, and moreover, if the patient forgets to change containers at night, he is unable to wear the lenses in the morning.

The aim of the present invention is to provide an improved method for cleaning and disinfecting contact lenses.

In accordance with the present invention, there is provided a method for cleaning and disinfecting a contact lens, wherein the lens is contacted for an extended period of up to 10 hours, for example, 5 to 10 hours, with a solution containing hydrogen peroxide, the contacting being effected in the presence of a solid, sustained-release matrix composition containing a peroxide inactivator which is gradually leached out of the matrix over the extended period, the matrix maintaining its integrity over the extended period and liberating sufficient peroxide inactivator such that substantially no peroxide remains in contact with the lens at the end of the extended period. The lens may then be contacted with a contact lens wetting or comfort solution before insertion into

EP 0 147 100 B2

the eye.

The prophylactic method of this invention is suited for adoption by the wearers of contact lenses as part of a regular maintainance régime. Soft, flexible, hard or other types of lenses can be cleaned and disinfected. Unlike the method of GB-A-1,472,409, heating or other extreme measures do not form part of the preventative method of this invention, since it is designed for continuing use before problematic deposits can build up on the lens.

The hydrogen peroxide cleaning and disinfecting solution preferably contains 0.1 to 5%, more preferably 0.3 to 1% of hydrogen peroxide, and may contain other components such as wetting agents and inorganic salts.

The sustained realease matrix composition suitably contains as peroxide inactivator agent which is gradually leached out of the matrix over the extended period. The composition preferably takes the form of a tablets, and because the composition maintains its integrity the lens may be easily separated from the treatment system. In this way the risk of sharp particles arising from a disintegrating sustained release composition is avoided.

The hydrogen peroxide solution is preferably used at the level of 2 to 15 ml, more preferably 5 to 15 ml, for example about 10 ml, per lens, along with 150 to 450 mg, for example about 300 mg, of sustained release composition.

The lenses is usually overnight at room temperature with hydrogen peroxide solution in the presence of the sustained release matrix composition. An indicator can be included in the solution/matrix system so that the user may visually determine that the end of the extended period has been reached. At the end of the containing there is substantially no hydrogen peroxide left in contact with the lens. Thus, the lens can be inserted directly into the eye or rinsed with a wetting or soaking solution and then inserted.

The present method can be used on one more lenses with available lens equipment, including the customary lens containers used for treating lenses with other cleaning solutions. However, it is particularly preferred to employ a novel cell comprising a base and a lid which can be together with a fluidtight join, the base having a rounded internal surface similar to of a contact lens.

In use of the cell, a contact lens can be placed in the rounded surface of the base along with the hydrogen peroxide solution and the sustained composition. The hydrogen peroxide solution can be put in the base with the lens and sustained release composition before the lid is secured. In a preferred construction, there is a mark, line or other indicator to indicate the appropriate level of liquid. For preference, the base and the lid each have a rounded internal surface of curvature similar to that of a contact lens to prevent adhesion of rigid lenses to the cell surfaces.

The present invention is illustrated by the following non-limiting examples.

### Example of Preparation of Sustained Release Tablets

Formulation I

| | Wt for 1000 tablets (g) |
|---|---|
| Sodium sulphite B.P.C. | 260 |
| Carnauba wax | 6 |
| Stearic acid BPC | 24 |
| Magnesium stearate BP | 1.25 |
| | 291.25 |

The sodium sulphite powder was anhydrous SLR grade. the powder passed through a No 60 sieve (i.e. less than 160 μm) and in general, was not retained on a No 85 sieve (i.e. less than 120 μm).

To determine the absorbancy of the sodium sulphite, chloroform was added, a few ml at a time, to 260 g of sodium sulphite in a mortar. This was continued until the mixture resembled a 'crumble-type' texture and until it retained its shape when compressed in the hand. At this point it was also possible to break the formed shape in half and crumble the section so formed. It was found that 60 ml chloroform was required to achieve this point.

The carnauba wax and stearic acid were weighed out into a breaker and made up to the 75 ml mark with chloroform (i.e. about 60 ml added). The mixture was heated in a water bath to dissolve the wax, and used while still hot before the wax solidified.

The resultant solution was then added to the sodium in a mortar and mixed in thoroughly with a pestle. A

3

spatula was used to break up the mixture, to allow some of the chloroform to evaporate, and thus obtain the correct texture.

The resultant waxy mass was put through a No 10 sieve, using hand pressure. The sieved granules were then weighed and then dried in a fluid bed dryer for about 10 minutes at about 40°C so that the wax did not melt. When no further chloroform could be detected from the granules they were weighed.

Weight of moist mass granules = 293.8 g

Weight of dry mass granules = 258.5 g

The dry granules were passed through a No. 16 sieve, again using hand pressure only, and poured into a polyethylene sample bag. The magnesium stereate was added to the contents of the bag and shaken thoroughly to ensure that it was evenly distributed throughout the granules.

Formulation II:

|  | Wt for 1000 tablets (g) |
| --- | --- |
| Sodium sulphite B.P.C. | 260 |
| Carnauba wax | 1.5 |
| Stearic acid BPC | 24 |
| Magnesium stearate BP | 1.25 |
|  | 286.75 |

Tablets were prepared in the same way as for Formulation I.

Example of Regime for Use of Present Method

The following is a suitable regime to be observed daily for cleaning and disinfecting lenses using the tablets and 0.6% v/v solution of hydrogen peroxide.

Stage I: Place one tablet in a container.

Stage II: Place one lens in the container.

Stage III: Add 10 ml of 0.6% v/v hydrogen peroxide solution to the container.

Stage IV: Close the container.

Stage V: Allow lens to remain immersed for at least 6 hours.

Stage VI: Remove the lens, rinse with a wetting or comfort solution if required.

Stage VII: Insert lens in the eye.

The order of the stages I to III may be varied.

## Claims

1. A method for cleaning and disinfecting a contact lens, wherein the lens is contacted for an extended period of up to 10 hours with a solution containing hydrogen peroxide, the contacting being effected in the presence of a solid, sustained-release matrix composition containing a peroxide which is gradually leached out of the matrix over the extended period, the matrix maintaining its integrity over the extended period and liberating sufficient peroxide inactivator such that substantially no peroxide remains in contact with the lens at the end of the extended period.

2. A method according to claim 1, wherein the hydrogen peroxide solution contains 0.1 to 5% hydrogen peroxide.

3. A method according to any preceding claim, wherein the hydrogen peroxide solution further contains wetting agents and/or inorganic salts.

4. A method according to any preceding claim, wherein the hydrogen peroxide solution is used at a level fo 2 to 15 ml per lens.

5. A method according to any preceding claim, wherein the sustained-release matrix composition is used at a level of 150 to 450 mg per lens.

6. A method according to any preceding claim, wherein the sustained-release matrix composition contains as peroxide inactivator a reducing agent.

7. A method according to any preceding claim, wherein the sustained-release matrix composition takes the form of a tablet.

8. A method according to any preceding claim, wherein an indicator is included in the solution/matrix system so that user may visually determine that the end of the extended period has been reached.

9. A solid, sustained-release matrix composition which slowly liberates a peroxide inactivator in hydrogen peroxide solution and which maintains its integrity over an extended period of up to 10 hours, the matrix composition taking the form of a tablet.

**Patentansprüche**

1. Verfahren zur Reinigung und Desinfizierung einer Kontaktlinse, wobei die Linse über eine längere Zeitdauer von bis zu 10 Stunden mit einer Wasserstoffperoxid enthaltenden Lösung in Kontakt gebracht wird, wobei der Kontakt in Gegenwart einer festen Matrixzusammensetzung mit Langzeit-Abgabe erfolgt, die einen Peroxidinaktivator enthält, der nach über die längere Zeitdauer aus der Matrix ausgelaugt wird, wobei die Matrix ihre Integrität Über die längere Zeitdauer aufrechterhält und ausreichend Peroxidinaktivator freisetzt, so daß am Ende der längeren Zeitdauer im wesantlichen kein Peroxid mit der Linse in Kontakt bleibt.

2. Verfahren nach Anspruch 1, worin die Wassertoffperoxidlösung 0,1 bis 5% Wasserstoffperoxid erhält.

3. Verfahren nach einem der vorstehend genannten Ansprüche, worin die Wasserstoffperoxidlösung weiters Benetzungsmittel und/oder anorganische Salze enthält.

4. Verfahren nach einem der vorstehend genannten Ansprüche, worin die Wasserstoffperoxidlösung in einer Menge von 2 bis 15 ml pro Linse verwendet wird.

5. Verfahren nach einem der vorstehend genannten Ansprüche, worin die Matrixzusammensetzung mit Langzeit-Abgabe in einer Menge von 150 bis 450 mg pro Linse verwendet wird.

6. Verfahren nach einem der vorstehend genannten Ansprüche, worin die Matrixzusammensetzung mit Langzeit-Abgabe als Peroxidinaktivator ein Reduktionsmittel enthält.

7. Verfahren nach einem der vorstehend genannten Ansprüche, worin die Matrixzusammensetzung mit Langzeit-Abgabe die Form einer Tablette hat.

8. Verfahren nach einem der vorstehend genannten Ansprüche, worin ein Indikator in dem System Matrix/Lösung inkludiert ist, so daß der Benutzer visuel erkennen kann, daß das Ende der längeren Zeitdauer erreicht ist.

9. Feste Matrixzusammensetzung mit Langzeit-Abgabe, die langsam in Waserstoffperoxidlösung einen Peroxidinaktivator freisetzt und ihre Integrität über längere Zeitdauer von bis zu 10 Stunden aufrechterhält, wobei die Matrixzusammensetzung die Form einer Tablette hat.

**Revendications**

1. Procédé de nettoyage et de désinfection de lentille de contact, dans lequelle la lantille est mise en contact pendant un intervalle de temps prolongé pouvant atteindre 10 heures, avec une solution contenant du peroxyde d'hydrogène, le contact étant réalisé en présence d'une composition de matrice solide à émission progressive contenant un désactivateèr de peroxyde, qui est extrait progressivement par dissolution de la matrice pendant l'intervalle de temps prolongé, la matrice conservant son intégrité sur tout l'intervalle de temps prolongé et libérant suffisamment de désactiveur de peroxyde de sorte qu'il ne reste sensiblement pas de peroxyde en contact avec la lentille à la fin de l'intervalle de temps prolongé.

2. Procédé selon la revendication 1, dans lequel la solution de peroxyde d'hydrogène contient 0,1 à 5% de peroxyde d'hydrogène.

3. Procédé selon l'une quelconque des revendications précédentes, dans lequel la solution de peroxyde d'hydrogène contient en outre des agents mouillants et/ou des sels minéraux.

4. Procédé selon l'une quelconque des revendications précédentes, dans lequel la solution de peroxyde d'hydrogène est utilisée à raison de 2 à 15 ml par lentille.

5. Procédé selon l'une quelconque des revendications précédentes, dans lequel la composition de matrice à émission progressive est utilisée à raison de 150 à 450 mg par lentille.

6. Procédé selon l'une quelconque des revendications précédentes, dans lequel la composition de matrice à émission progressive contient comme désactivateur de peroxyde un agent réducteur.

7. Procédé selon l'une quelconque des revendications précédentes, dans lequel la composition de matrice à émission progressive a la forme d'un comprimé.

8. Procédé selon l'une quelconque des revendications précédentes, dans lequel un indicateur est inclus dans le système solution/matrice de sorte que l'utilisateur peut déterminer visuellement quand la fin de l'intervalle de temps prolongé a été atteint.

9. Composition de matrice solide à émission progressive qui libère un désactivateur de peroxyde dans la solution de peroxyde d'hydrogène et qui maintient son intégralité sur un intervalle de temps prolongé, jusqu'à 10 heures, la composition de matrice prenant la forme d'un comprimé.